# EUROPEAN PATENT APPLICATION

(11) **EP 1 602 774 A1**
(43) Date of publication of application: **07.12.2005**
(21) Application number: 05102087.3
(22) Date of filing: 16.03.2005
(51) Int. Cl.: D06F 58/20, D06F 25/00

(54) **Washing machine**

(30) Priority: 31.05.2004 KR 2004039315
(71) Applicant: SAMSUNG ELECTRONICS CO., LTD., Suwon-si, Gyeonggi-Do 442-742 (KR)
(72) Inventor: Yang, Hye Soon, Yongin-Si Gyeonggi-Do (KR); Yang, Byoung Yull 905-1103 Greenvil Jookong 9th, Ansan-Si Gyeonggi-Do (KR); Oak, Seong Min, Gyeongsangnam-Do (KR); Park, Jae Ryong, Youngtong-Gu Suwon-Si Gyeonggi-do (KR); Pyo, Sang Yeon, Youngtong-Gu Suwon-Si Gyeonggi-do (KR); Kim, Hyung Gyoon, Youngtong-Gu Suwon-Si Gyeonggi-do (KR)
(74) Representative: Grey, Ian Michael

(57) **Abstract**

A washing machine, which removes offensive odour from laundry without operating wash, rinse and/or dehydration modes, and a deodorizing method thereof. The washing machine includes a deodorizing tub (21), into which objects to be deodorized are put, a duct (30) communicating with the deodorizing tub (21) and serving as a passage for air containing odour particles separated from the objects in the deodorizing tub, and a deodorizing unit (50) for removing the odour particles from the air passing through the duct (30). The deodorizing method includes separating odour particles from objects to be deodorized, and removing the separated odour particles by filtering out the separated odour particles with a deodorizing filter (52).

## Description

The present invention relates to a washing machine comprising a tub to receive laundry, an airflow duct having an inlet in communication with the inside of the tub and means to draw air from the tub into the air flow duct.

A drum-type washing machine washes laundry contained in a drum by rotating the drum and agitating the laundry in detergent and water. Compared to a conventional pulsator-type washing machine, this type of washing machine has a long washing time but reduces water consumption and damage to the washing machine. As a result the use of a drum-type washing machine is increasingly common.

A conventional drum-type washing machine has various modes such as wash, rinse, dehydration and dry. Using a conventional drum washing machine to remove offensive odours, such as the smell of food or tobacco from laundry, requires the use of the wash mode thereby causing waste of electricity and water and requiring a long time to wash the laundry.

The present invention seeks to provide a system which overcomes or substantially alleviates the problems discussed above.

A drum-type washing machine according to the present invention is characterised by filtration means located in the airflow duct to remove odour particles from the air flow.

Preferably, the filtration means comprise an air filter located in a holder, an inlet to allow unfiltered air to enter and an outlet located substantially at an opposite end to the inlet.

In a preferred embodiment, the outlet exhausts air to the exterior of the washing machine.

In one embodiment, the filter material is one of either a ceramic filter or an activated carbon filter.

The washing machine may further comprise ultraviolet ray producing means, such as UV-LED lamps located to irradiate the air filter.

Preferably, the deodorising filter is coated with a photo catalyst.

Preferably, the filter material is removable from the filter material holder to be replaced or cleaned.

In one embodiment, the washing machine further comprises means of controlling the airflow to the filter.

The means to control the flow of air to the filter may include a damper and a damper-driving motor to control the damper position.

A method of removing odour particles from the air in a washing machine cycle is characterised by the step of filtering the air in the airflow path to remove odour particles from the airflow.

Preferably, the method further includes the step of exhausting the air to the exterior of the washing machine.

In a preferred embodiment, the washing machine further comprises ultraviolet producing means, in the form of UV-LED's, the method further including the steps of producing UV rays, irradiating the filter, decomposing the odour particles through the means of photocatalytic action.

Embodiments of the present invention will be described, by way of example only, with reference to the accompanying drawings, in which:
Figure 1 is a perspective view of a washing machine having a deodorizing filter in accordance with a preferred embodiment of the present invention;
Figure 2 is a longitudinal sectional view of the washing machine of Figure 1;
Figure 3 is an exploded perspective view of the deodorizing filter of the washing machine of Figure 1; and
Figure 4 is a schematic view illustrating a deodorizing operation of the washing machine of Figure 1.

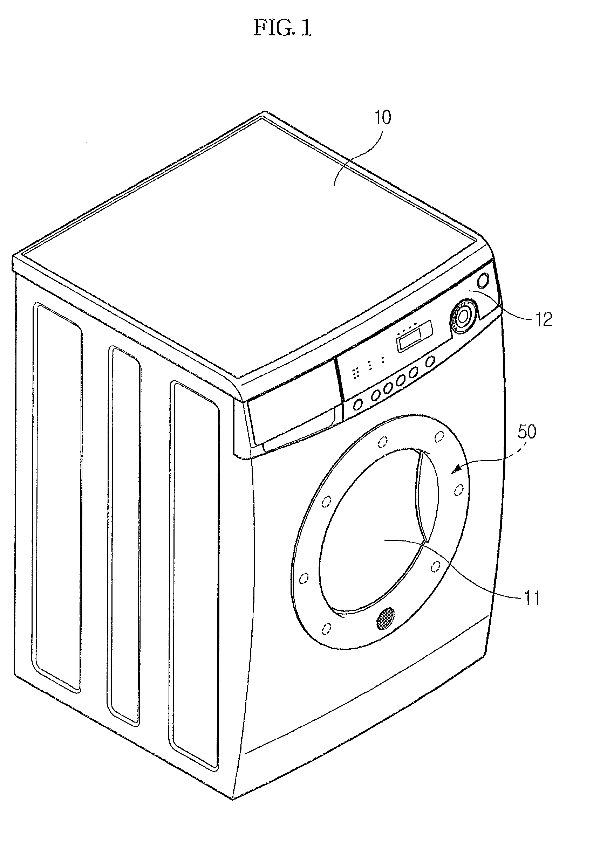

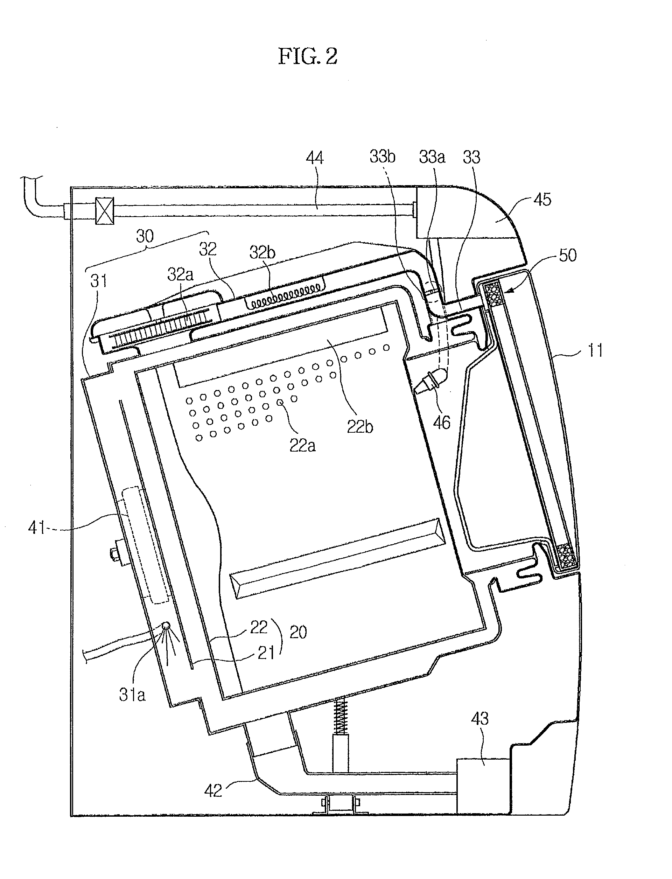

Referring to the drawings, there is shown in Figures 1 and 2 a washing machine of the present invention comprising a housing 10 defining the external appearance of the washing machine, a door 11, installed on a front surface of the housing 10, which opens and closes so that laundry to be deodorized may be placed into the washing machine and an operating unit 12 installed on the front surface of the housing 10 above the door 11 to determine the operation of the washing machine.

A cylindrical drum arrangement 20 wherein the end nearest the door is open, is placed in the housing 10. The drum arrangement 20 serves as a deodorizing tub during a deodorizing process, and has a double structure including a fixed tub 21 and a rotary tub 22. Here, the rotary tub 22 is rotatably installed in the fixed tub 21, and is operated by driving motor 41 installed on a rear surface of the fixed tub 21. The rotary tub 22 includes a plurality of through holes 22a formed through side and rear surfaces thereof so that the rotary tub 22 communicates with the fixed tub 21 and a plurality of lifters 22b to lift the laundry installed in a longitudinal direction on the inner circumference thereof.

A water feed pipe 44 is installed above the upper surface of the fixed tub 21 to supply water to the inside of the drum 20. One end of the water feed pipe 44 is connected to the rear surface of the housing 10 to receive water from an external water supply source (not shown), and the other end of the water feed pipe 44 is connected to a detergent container 45 installed in the front surface of the housing 10. A water feed nozzle 46 is connected to the detergent container 45 such that water supplied to the detergent container 45 is subsequently supplied to the inside of the drum 20. In order to discharge used water to the outside, a drain pipe 42 and a drain pump 43 are installed on the lower part of the fixed tub 21.

A duct 30 to recirculate air taken from the inside of the drum 20 is formed on the outer circumference of the fixed tub 21. The duct 30 is divided into a condensing duct 31, which is installed on the rear surface of the fixed tub 21 in a longitudinal direction and communicates with a lower part of the fixed tub 21, and a blowing duct 32, which communicates with the upper end of the deodorizing duct 31, extends along the upper surface of the fixed tub 21, and then communicates with the inside of the drum 20 at the upper part of the front surface of the fixed tub 21.

The condensing duct 31 condenses moisture in the high temperature and high humidity introduced into the duct 31 from the drum 20 during the dehydration mode provided with a condensate water nozzle 31a to spray cold water to the air passing through the condensing duct 31. The blowing duct 32 is provided with an air fan 32a to circulate air between the drum 20 and the duct 30, and a heater 32b to heat the air that is introduced into the drum 20.

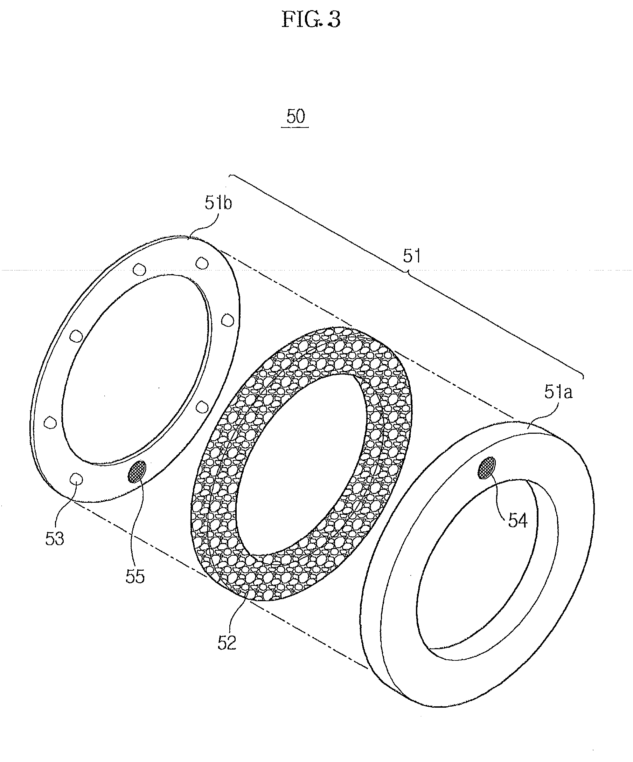

A deodorizing unit 50 having a circular loop shape is installed along the circumference of a transparent window formed in the door 11 which allows the inside of the drum 20 to be viewed. The deodorizing unit 50 eliminates odour particles in the air that are separated from objects to be deodorized. A detailed structure of the deodorizing unit 50 is shown in Figure 3.

The deodorizing unit 50 includes an annular filter case 51 and a deodorizing filter 52 installed in the filter case 51.

A conventionally well known filter, such as a ceramic filter or an activated carbon filter, is used as the deodorizing filter 52. The deodorizing filter 52 serves to remove odour particles from the air flow. The deodorizing filter 52 is coated with a photocatalyst, such as titanium oxide (TiO₂). When ultraviolet rays are irradiated on the photocatalyst, the photocatalyst, which has a high oxidising power, oxidizes a contaminant that is adhered to the objects to be deodorized. As a result, the contaminant is decomposed. This is generally referred to as a "photocatalytic action". The oxidation of the contaminant by the photocatalyst lengthens the lifespan of the deodorizing filter 52. The ultraviolet rays, which have a strong bactericidal action, prevent contamination that would be generated due to the propagation of micro-organisms, which may be dispersed from the deodorizing filter 52 so as to maintain a cleanliness of the deodorizing filter 52.

The filter case 51 includes a main body 51a, into which the deodorizing filter 52 is inserted, and a front cover 51b that is detachably attached to the main body 51a. UV-LED lamps 53 to irradiate ultraviolet rays are installed on the inner surface of the front cover 51b. The front cover 51b is made of a semi-transparent plastic.
This allows a user to view the oxidation of the contaminant by the ultraviolet rays irradiated from the UV-LED lamps 53 through the front cover 51b. An inlet 54, through which air is introduced from the blowing duct 32 into the filter case 51, is formed through the rear surface of the upper portion of the main body 51a. An outlet 55, through which air filtered by the deodorizing filter 52 is exhausted to the outside of the washing machine therethrough, is formed through the lower portion of the front cover 51b. A filter net to filter out comparatively large sized particles of the contaminant is installed in each of the inlet 54 and the outlet 55.

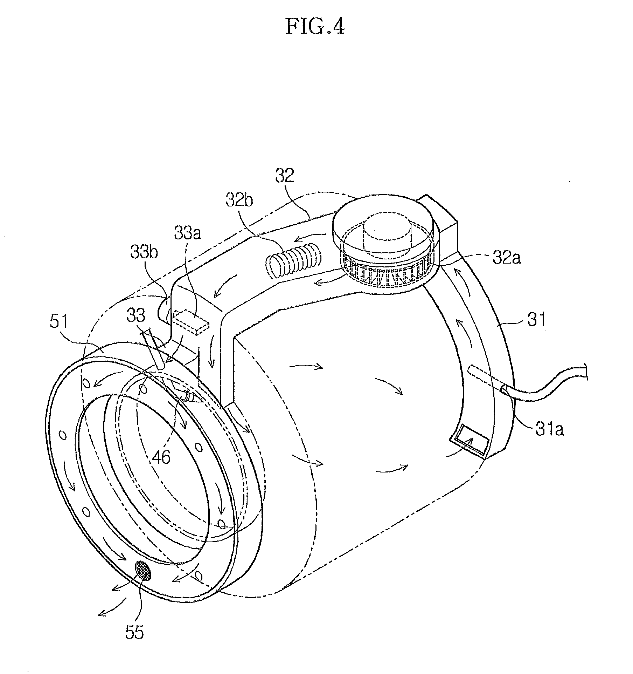

Now, with reference to figure 4, the structure of a branch duct 33 will be described. In order to circulate the air containing odour particles into the deodorizing unit 50, a branch duct 33 in communication with the inlet 54 of the filter case 51 is branched from the front end of the blowing duct 32. A damper 33a, to control the flow of air into the branch duct 33 to be opened only in the deodorization mode, is installed in the branch duct 33, and a damper-driving motor 33b, to drive the damper 33a, is installed outside the branch duct 33.

Now, a deodorizing method of the washing machine having the deodorizing filter in accordance with the above embodiment of the present invention will be described.

Schematically, after particles generating offensive odour, such as the smell of food, tobacco, etc. are separated from objects permeated by the offensive odour, air containing the separated odour particles passes through the deodorizing unit 50, so that the deodorizing filter 52 can filter out odour particles.

Methods of separating the odour particles from the objects to be deodorized are as follows. A first method involves mechanically agitating the objects to be deodorized. In the first method, when the rotary tub 22 containing the objects is rotated, the odour particles are separated from the objects by agitation, such as the frictional force between the objects or between the objects and the rotary tub 22 and the impact generated by the falling of the objects in the rotary tub 22. A second method includes applying heat to the objects to be deodorized. According to the second method, when the heater 32b is operated in conjunction with the air fan 32b, a hot draft heated by the heater 32b is supplied to the inside of the drum 20. The hot draft contacts the objects to be deodorized, and induces movement of the odour particles stuck to the objects so as to separate the odour particles from the objects. A third method is that odour particles that permeate the objects to be deodorized are dissolved in water. According to the third method, when a small quantity of water in mist form is supplied to the inside of the drum 20 through the water feed nozzle 46, the odour particles stuck to the objects are dissolved in water and then are vaporized together with the water so as to be separated from the objects. Combinations of the above three methods may be selectively used combined so as to improve the deodorizing effect. Since the odour particles permeating the objects to be deodorized have different adhesive strengths to the objects based on the type of odour particle, in an embodiment of the invention, the above methods are suitably combined based on the adhesive strengths of the odour particles to the objects.

The odour particles separated from the objects by the above methods float in the drum 20. In order to prevent the separated odour particles from being re-adhered to the objects, the separated odour particles are discharged to the outside of the drum 20.

When the air blast fan 32a is operated, air containing the odour particles in the drum 20 is circulated through the duct 30. As shown by the arrows in figure 4, air containing the odour particles, which is drawn into the condensing duct 31 is introduced into the blowing duct 32 so that the air passes through the air blast fan 32a and the heater 32b and enters into the upper portion of the front part of the inside of the fixed tub 21 communicating with the blowing duct 32. Here, a part of the air, having passed through the blowing duct 32, flows towards the branch duct 33, and when the damper driving motor 33b is driven to open the damper 33a, the above part of the air is drawn into the deodorizing unit 50. The odour particles of the air drawn into the deodorizing unit 50 are filtered by the deodorizing filter 52, and the purified air is exhausted to the outside of the washing machine through the outlet 55.

When the above part of the air circulated in the drum 20 is exhausted to the outside of the washing machine, the inside of the drum 20 has a pressure that is lower than that of the outside of the drum 20. Thus, the inside of the drum 20 is filled with new air through gaps formed along the circumference of the drum 20. The gaps allow the new air to compensate for the lowered pressure of the inside of the drum 20. The remainder of the air which has not passed through the branch duct 33, is again drawn into the drum 20 and recirculated in the drum 20. As described above, most of the odour particles, which are separated from the objects to be deodorized, are filtered out by the deodorizing filter 52.

In a case that the UV-LED lamps 53 are operated in the deodorization mode, the deodorizing filter 52 performs a photocatalytic action to oxidize the odour particles and other contaminants. Where the deodorizing filter 52 loses deodorizing capacity due to the use of the deodorizing filter 52 over a long period of time, the deodorizing filter 52 is either replaced with a new deodorizing filter of may be washed and reused. Whether the deodorizing filter 52 is replaced or washed and reused is dependent on the type of deodorizing filter. Where the deodorizing filter 52 is an activated carbon filter, the deodorizing filter 52 should be replaced. Meanwhile, where the deodorizing filter 52 is a ceramic filter, the deodorizing filter should be simply washed and reused.

Although the separation of the odour particles from the objects to be deodorized and the removal of the separated odour particles using the deodorizing filter were sequentially described in the above noted embodiment of the present invention for convenience, the two operations are substantially performed simultaneously.

As is apparent from the above description, the present invention provides a washing machine having a deodorizing filter, which removes particles of offensive odour from laundry without operating wash, rinse and/or dehydration modes, and a deodorizing method thereof.

Thereby, the washing machine and the deodorizing method of the present invention reduced electricity and water consumption and prevent the deformation of the laundry generated by the washing.

Although embodiments of the present invention have been shown and described, it would be appreciated by those skilled in the art that changes may be made to these embodiments without departing from the principles of the invention, the scope of which is defined in the claims and their equivalents and the foregoing description should be regarded as a description of a preferred embodiment only.

## Claims

1. A washing machine comprising a tub to receive laundry, an airflow duct having an inlet in communication with the inside of the tub and means to draw air from the tub into the airflow duct **characterised by** filtration means associated with the airflow duct to remove odour particles from the air flow.

2. A washing machine according to claim 1 wherein the filtration means comprise a deodorising filter located in a housing having an inlet to allow unfiltered air to enter the filter from the duct and an outlet spaced from the inlet for the passage of air out of the filtration means.

3. A washing machine according to claim 2 wherein the filter housing is annular in shape and is disposed so that it surrounds the periphery of an opening in the tub through which laundry is placed into and removed from the tub.

4. A washing machine according to claim 2 or 3 wherein the deodorizing filter is one of either a ceramic filter or an activated carbon filter.

5. A washing machine according to any of claims 2 to 4 wherein the outlet exhausts air to the exterior of the washing machine.

6. A washing machine according to any of claims 2 to 5 further comprising ultraviolet ray producing means, such as UV-LED lamps, located in the housing so as to irradiate the deodorising filter.

7. A washing machine according to claim 6 wherein the deodorising filter is coated with a photocatalyst.

8. A washing machine according to any of claims 2 to 7 wherein the deodorising filter is removable from the housing for replacement or cleaning.

9. A washing machine according to any preceding claim wherein the airflow duct has a return path to the tub and a branch duct to the filtration means.

10. A washing machine according to claim 8, further comprising a damper and a damper-driving motor to control the damper position located in the branch duct to selectively control the flow of air through the return path and the branch duct.

11. A method of controlling a washing machine to remove odour particles from the air in a tub that receives laundry comprising the step of drawing air from the tub through an airflow duct having an inlet in communication with the inside of the tub so that it passes through a filter associated with the duct to remove odour particles from the airflow.

12. A method of removing odour particles from the air in a washing machine cycle according to claim 11, wherein the method further includes the step of exhausting the air to the exterior of the washing machine.

13. A method of removing odour particles from the air in a washing machine cycle according to claim 11, the washing machine further comprising ultraviolet producing means, in the form of UV-LED's, the method further including the steps of producing UV rays, irradiating the filter, decomposing the odour particles through the means of photocatalytic action.

14. A washing machine comprising a deodorizing tub, into which objects to be deodorized are put, a duct to communicate with the deodorizing tub and to serve as a passage through which air containing odour particles, which are separated from the objects in the deodorizing tub passes, and a deodorizing unit to remove the odour particles from the air passing through the duct.

15. A washing machine comprising a deodorizing tub into which objects to be deodorized are put, a duct to communicate with the deodorizing tub and to serve as a passage through which air containing odour particles, which are separated from the objects in the deodorizing tub passes, and a deodorizing unit including a deodorizing filter to filter out and remove the odour particles from the air passing through the duct.

16. The washing machine according to claim 15 wherein the deodorizing unit further includes a photocatalyst to perform a photocatalytic action so as to oxidize the odour particles in the deodorizing filter and UV-LED lamps.

17. The washing machine according to claim 15 wherein the duct, in communication with the deodorizing tub, circulates the air inside the deodorizing tub and a branch duct is branched from the duct such that a part of the circulated air passes through the deodorizing unit and communicates with the deodorizing unit.

18. The washing machine according to claim 17 wherein the branch duct is provided with a damper to open and close the branch duct.

19. The washing machine according to claim 17 wherein the deodorizing unit further includes a filter case to form a passage for the air flowing along the deodorizing filter so that the air containing the odour particles introduced from the branch duct is purified by the deodorizing filter and the filter case is provided with an outlet, through which the air purified by the deodorizing filter is exhausted to the outside of the washing machine.

20. The washing machine according to claim 19 wherein the deodorizing unit is installed on a door of the washing machine.

21. The washing machine according to claim 17 wherein the duct includes a condensing duct to communicate with a rear surface of the deodorizing tub and a blowing duct to communicate with the condensing duct to be positioned on an upper surface of the deodorizing tub, the blowing duct is provided with an air blast fan to generate power so as to circulate the air passing through the blowing duct, and a heater to heat the air passing through the blowing duct and the branch duct is branched from the blowing duct.

22. The washing machine according to claim 15 wherein the deodorizing filter is a ceramic filter or an activated carbon filter.

23. The washing machine according to claim 16 wherein the deodorizing filter is coated with the photocatalyst.

24. A deodorizing method of a washing machine comprising separating odour particles from objects to be deodorized and removing the separated odour particles by filtering out the separated odour particles with a deodorizing filter.

25. The deodorizing method according to claim 24 wherein the separating comprises at least one of supplying a hot draft to the objects to be deodorized, applying mechanical impact to the objects to be deodorized, or dissolving the odour particles in moisture that is supplied to the objects to be deodorized in a mist.

26. The deodorizing method according to claim 19 wherein in the removing, the air in the deodorizing tub, in which the separated odour particles float, is circulated into a duct communicating with the deodorizing tub, and a part of the circulated air containing the separated odour particles are purified by the deodorizing filter.

27. The deodorizing method according to claim 26 further comprising performing a photocatalytic action to oxidize the odour particles in the deodorizing filter.

28. The deodorizing method according to claim 27 further comprising exhausting the air that is purified by the deodorizing filter to the outside of the washing machine.

29. A washing machine including a deodorizing tub having a fixed tub and a rotary tub into which objects to be deodorized are placed comprising a duct to communicate with the deodorizing tub through which air containing odour particles, which are separated from the objects in the deodorizing tub passes and a deodorizing unit to filter out and remove the odour particles from the air passing through the duct.

30. A washing machine including a deodorizing tub having a fixed tub and a rotary tub into which objects to be deodorized are placed comprising a duct to communicate with the deodorizing tub through which air containing odour particles which are separated from the objects in the deodorizing tub passes, a branch duct, branched from the duct, through which a part of the circulated air that passes through the duct passes, and a deodorizing unit to filter out and remove the odour particles from the air which passes through the branch duct.

31. A deodorizing method of a washing machine comprising separating odour particles from objects to be deodorized such that the odour particles are carried in an air flow, and removing the odour particles by filtering the air flow with a deodorizing filter.

32. A washing machine including a deodorizing tub having a fixed tub and a rotary tub into which objects to be deodorized are placed comprising a duct to communicate with the deodorizing tub through which air containing odour particles, which are separated from the objects in the deodorizing tub passes, and a deodorizing unit to filter out and remove the odour particles from the air passing through the duct, wherein the odour particles are separated and removed substantially simultaneously.

33. A deodorizing method of a washing machine comprising separating odour particles from objects to be deodorized such that the odour particles are carried in an air flow and removing the odour particles by filtering the air flow with a deodorizing filter wherein the separating and the removing are performed substantially simultaneously.
